# FASCICULE DE BREVET EUROPEEN

(11) **EP 4 429 749 B1**
(45) Date de publication et mention de la délivrance du brevet: **16.04.2025**
(21) Numéro de dépôt: 22830867.2
(22) Date de dépôt: 07.12.2022
(51) Int. Cl.: A61M 39/02, A61J 15/00, A61M 39/10

(54) **CAPUCHON POUR UN ENSEMBLE DE VERROUILLAGE D'UN SYSTÈME D'ALIMENTATION ENTÉRALE**
KAPPE FÜR EINE VERRIEGELUNGSANORDNUNG EINES ENTERALEN ERNÄHRUNGSSYSTEMS
CAP FOR A LOCKING ASSEMBLY OF AN ENTERAL FEEDING SYSTEM

(30) Priorité: 07.12.2021 FR 2113096
(43) Date de publication de la demande: 18.09.2024
(73) Titulaire: Tremma, 64140 Billère (FR)
(72) Inventeur: MERCIER, Anne, 64140 Billère (FR); CABAUD, Louis-Marie, 64140 Billère (FR); KOUTIRI, Imade, 64140 Billère (FR); RICHAUD, Emmanuel, 64140 Billère (FR)
(74) Mandataire: A.P.I. Conseil
(86) Numéro de dépôt international: PCT/EP2022/084874
(87) Numéro de publication internationale: WO 2023/104930

(56) Documents cités:
- WO-A1-02/066108
- WO-A1-2016/007166
- US-A1- 2012 053 525
- US-A1- 2014 155 839

## Description

### Domaine technique

L'invention concerne le domaine des connectiques de nutrition entérale. En particulier, elle concerne un capuchon pour un ensemble de verrouillage d'un système d'alimentation entérale. Des dispositifs similaires sont connus des documents WO02066108A1, US2012053525A1, WO2016007166A1 et US2014155839A1.

### Technique antérieure

On connait des mécanismes de verrouillage de système d'alimentation entérale que l'on verrouille par rotation de deux éléments de verrouillages complémentaires, l'un par rapport à l'autre.

Or, certains porteurs de systèmes d'alimentation entérale, tels que les enfants, bougent beaucoup lorsque le système d'alimentation entérale est en place ; en particulier, la nuit.

Dans ce cas, les nombreux mouvements réalisés par le porteur du système d'alimentation entérale peuvent involontairement provoquer le passage d'un état verrouillé à un état déverrouillé de l'ensemble de verrouillage.

Une telle situation peut avoir de graves conséquences sur la santé du porteur du système d'alimentation entérale.

Ainsi, il existe un besoin pour une solution qui permet d'éviter la situation décrite ci-dessus.

### Résumé de l'invention

L'invention vise à résoudre, au moins partiellement, ce besoin.

L'invention vise en particulier un capuchon pour un ensemble de verrouillage d'un système d'alimentation entérale.

En particulier, l'ensemble de verrouillage comprend une base d'un dispositif de cathéter et un raccord adapté pour être raccordé de manière amovible à la base de sorte que le raccord et la base sont verrouillés l'un par rapport à l'autre, en direction verticale et horizontale, l'ensemble de verrouillage étant configuré pour être verrouillé/déverrouillé par une rotation du raccord par rapport à la base.

En pratique, la base présente une première partie d'extrémité, une partie centrale et une deuxième partie d'extrémité qui est opposée à la première partie d'extrémité, la première partie d'extrémité et la deuxième partie d'extrémité faisant saillie depuis la partie centrale, la base ayant une surface supérieure et une surface inférieure, la surface supérieure comprenant un premier élément de verrouillage.

Par ailleurs, le raccord comprend un corps de raccord et une conduite, la conduite définissant un chemin de fluide et étant couplée à un tube, le corps de raccord comprenant une surface supérieure et une surface inférieure, la surface inférieure comprenant un deuxième élément de verrouillage qui présente une forme complémentaire à celle du premier élément de verrouillage et qui est configuré pour évoluer entre une configuration d'engagement et une configuration de verrouillage.

En pratique, dans la configuration d'engagement, le premier élément de verrouillage et le deuxième élément de verrouillage entrent en prise l'un avec l'autre.

Par ailleurs, dans la configuration de verrouillage, en réponse à une rotation du raccord par rapport à la base dans une direction de verrouillage prédéterminée, la conduite se trouve au-dessus d'une partie d'extrémité de la base, dite partie d'extrémité de verrouillage, et le premier élément de verrouillage et le deuxième élément de verrouillage sont en prise l'un avec l'autre de sorte que le raccord et la base sont verrouillés l'un par rapport à l'autre, en direction verticale et horizontale.

Dans l'invention, le capuchon comprend un corps de capuchon en matériau élastiquement déformable de manière à se déformer sous une force et revenir à un état initial non déformé après la suppression de la force, le corps de capuchon comprenant un corps de capuchon ayant une paroi supérieure et une première paroi verticale qui supporte la paroi supérieure et qui est raccordée au niveau d'une extrémité arrière de la paroi supérieure.

En particulier, la première paroi verticale comprend une première ouverture de passage qui s'étend sur tout ou partie d'une largeur et/ou d'une hauteur de la première paroi verticale et qui,
- dans la configuration d'engagement, est traversée par le tube,
- dans la configuration de verrouillage, entoure étroitement, au moins, tout ou partie de la conduite et de la partie d'extrémité de verrouillage, de manière à verrouiller en rotation la base et le raccord l'un par rapport à l'autre.

En outre, la paroi supérieure comprend une surface intérieure qui définit un logement creux qui est formé dans une épaisseur de la paroi supérieure, de sorte que dans la configuration de verrouillage, le logement creux reçoit étroitement, par clipsage, tout ou partie de la surface supérieure du corps de raccord.

Par ailleurs, le corps de capuchon est agencé de sorte que sous l'effet des propriétés élastiques du corps de capuchon, la surface supérieure du corps de raccord pénètre dans le logement après que la première ouverture de passage entoure tout ou partie de la conduite et de la partie d'extrémité de verrouillage.

Dans un premier mode réalisation, le corps de capuchon présente une section en forme de L.

Dans une mise en œuvre du premier mode de réalisation, la paroi supérieure présente une surface extérieure qui comprend une portion de déclipsage qui fait saillie en porte-à-faux vers l'extérieur depuis une extrémité avant de la paroi supérieure et qui, dans la configuration de verrouillage, est configurée pour être inclinée manuellement sous l'effet d'une force exercée sur une surface inférieure de la portion de déclipsage pour entraîner le déclipsage de la surface supérieure du corps de raccord du logement.

Dans un deuxième monde de réalisation, la largeur de la première ouverture de passage, dans son état non déformé, est inférieure à une largeur de partie centrale de la base mode réalisation.

Dans un troisième monde de réalisation, la première paroi verticale est configurée pour que dans la configuration de verrouillage, une portion de la partie d'extrémité de verrouillage dépasse d'une longueur prédéterminée au-delà d'une face arrière de la première ouverture de passage.

Dans un quatrième monde de réalisation, la paroi supérieure et la première paroi verticale sont réalisées d'un seul tenant.

Dans un cinquième monde de réalisation, le capuchon comprend en outre une deuxième paroi verticale qui supporte la paroi supérieure et qui est raccordée au niveau d'une extrémité avant de la paroi supérieure de sorte que le corps de capuchon présente une section en forme de U, la deuxième paroi verticale comprenant une deuxième ouverture de passage qui s'étend sur tout ou partie d'une largeur et/ou d'une hauteur de la deuxième paroi verticale et qui,
- dans la configuration de verrouillage, entoure étroitement, au moins, la partie d'extrémité de la base qui est opposée à la partie d'extrémité de verrouillage, dite extrémité opposée, de manière à verrouiller encore davantage en rotation la base et le raccord l'un par rapport à l'autre

Par ailleurs, le corps de capuchon est agencé de sorte que la deuxième ouverture de passage ne peut entourer la partie d'extrémité opposée qu'après que la surface supérieure du corps de raccord pénètre dans le logement.

Dans une première mise en œuvre du cinquième monde de réalisation, la largeur de la deuxième ouverture de passage, dans son état non déformé, est inférieure à une largeur de partie centrale de la base.

Dans une deuxième mise en œuvre du cinquième monde de réalisation, la paroi supérieure, la première paroi verticale et la deuxième paroi verticale sont réalisées d'un seul tenant.

Dans une troisième mise en œuvre du cinquième monde de réalisation, la deuxième paroi verticale est configurée pour que dans la configuration de verrouillage, une portion de la partie d'extrémité opposée dépasse d'une longueur prédéterminée au-delà de la face avant de la deuxième ouverture de passage.

### Brève description des dessins

D'autres caractéristiques et avantages de l'invention seront mieux compris à la lecture de la description qui va suivre et en référence aux dessins annexés, donnés à titre illustratif et nullement limitatif.
[Fig.1] La [Fig.1] représente un ensemble de verrouillage d'un système d'alimentation entérale.
[Fig.2] La [Fig.2] représente une vue de coupe d'un capuchon selon l'invention.
[Fig.3] La [Fig.3] représente une vue de face du capuchon de la [Fig.2].
[Fig.4] La [Fig.4] représente une vue du dessus du capuchon de la [Fig.2].
[Fig.5] La [Fig.5] représente une vue du dessous du capuchon de la [Fig.2].
[Fig.6] La [Fig.6] représente le capuchon de la [Fig.2] dans la configuration d'engagement.
[Fig.7] La [Fig.7] représente le capuchon de la [Fig.2] en train de passer dans la configuration de verrouillage.
[Fig.8] La [Fig.8] représente le capuchon de la [Fig.2] dans la configuration de verrouillage.
[Fig.9] La [Fig.9] représente une mise en œuvre particulière d'un capuchon selon l'invention.

Les figures ne respectent pas nécessairement les échelles, notamment en épaisseur, et ce, à des fins d'illustration.

### Description des modes de réalisation

Le contexte dans lequel est mise en œuvre l'invention est celui d'un ensemble de verrouillage d'un système d'alimentation entérale.

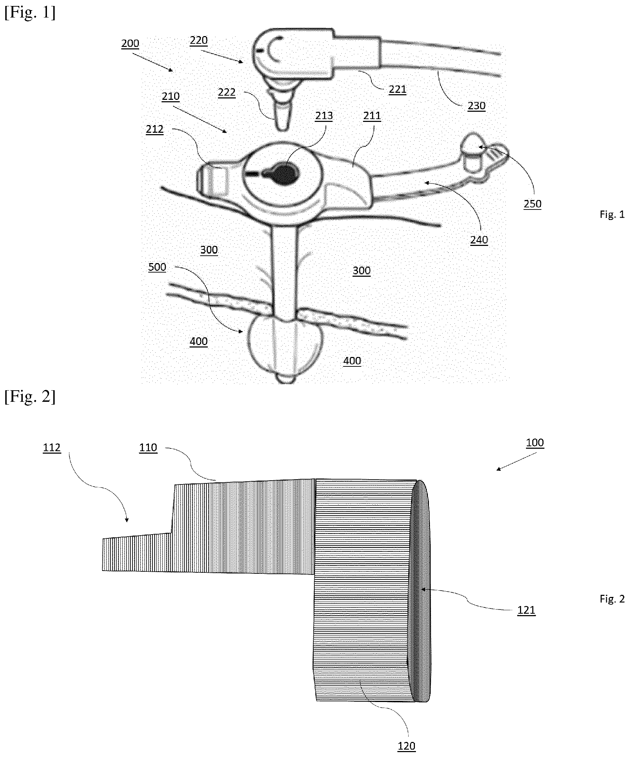

Dans l'exemple de la [Fig.1], le système d'alimentation entérale, est un système d'alimentation par gastrostomie.

Toutefois, selon les besoins, on pourra envisager d'utiliser d'autres aboutements chi-rurgicaux d'un organe creux avec la peau d'un porteur de système d'alimentation entérale, et ce, sans nécessiter de modifications substantielles de l'invention.

Dans l'exemple de la [Fig.1], et de manière connue, le système d'alimentation entérale est disposé en partie à travers la paroi abdominale d'un porteur du système d'alimentation entérale, au moyen d'un dispositif de cathéter qui traverse une couche de peau 300 du porteur, puis son estomac 400 avant de déboucher sur un ballonnet 500 gonflable.

Comme illustré sur la [Fig.1], le système d'alimentation entérale inclut un ensemble de verrouillage 200 qui comprend une base 210 du dispositif de nutrition et un raccord 220.

En particulier, la base 210 comprend une première partie d'extrémité 211, une partie centrale et une deuxième partie d'extrémité 212.

En pratique, la partie centrale est disposée entre la première partie d'extrémité 211 et la deuxième partie d'extrémité 212.

Par ailleurs, la première partie d'extrémité 211 présente une largeur de première partie d'extrémité, la partie centrale présente une largeur de partie centrale et la deuxième partie d'extrémité présente une largeur de deuxième partie d'extrémité.

Dans un exemple, la largeur de partie centrale est supérieure à la largeur de première partie d'extrémité et également supérieure à la largeur de deuxième partie d'extrémité.

De manière connue, la deuxième partie d'extrémité 212 se situe au niveau du site d'inflation (« inflation port », en anglais) du ballonnet 500.

En outre, la base 210 présente une surface supérieure et une surface inférieure.

On entend par « supérieur » et «inférieur» les parties situées respectivement en haut et en bas sur la représentation de la [Fig.1].

En particulier, la surface supérieure comprend un premier élément de verrouillage 213.

Dans le contexte de l'invention, le premier élément de verrouillage 213 se situe au niveau du site d'alimentation (« feeding port», en anglais) du système d'alimentation entérale.

Aussi, dans un exemple de la base 210, celle-ci comprend une languette 240 qui est raccordée à la première partie d'extrémité 211.

En outre, la languette 240 comprend un couvercle 250 qui est configuré pour fermer le site d'alimentation dans une première position et le libérer dans une deuxième position.

Comme illustré sur la [Fig.1], le raccord 220 comprend un corps de raccord et une conduite 221.

En particulier, la conduite 221 définit un chemin de fluide et est couplée à un tube.

Dans un exemple, le tube 230 est un tube d'alimentation en fluide médical.

Par ailleurs, le corps de raccord comprend une surface supérieure et une surface inférieure.

En particulier, la surface inférieure du corps de raccord comprend un deuxième élément de verrouillage 222 qui présente une forme complémentaire à celle du premier élément de verrouillage 213

En outre, le deuxième élément de verrouillage 222 est configuré pour évoluer entre une configuration d'engagement et une configuration de verrouillage.

Dans la configuration d'engagement, le premier élément de verrouillage 213 et le deuxième élément de verrouillage 222 entrent en prise l'un avec l'autre.

Ainsi, dans la configuration d'engagement, l'ensemble de verrouillage 200 est dans un état déverrouillé.

Dans un premier exemple de l'ensemble de verrouillage 200, le premier élément de verrouillage 213 comprend une ouverture de verrouillage et le deuxième élément de verrouillage 222 comprend une tige de verrouillage. Dans ce cas, dans la configuration d'engagement, l'ouverture de verrouillage reçoit la tige de verrouillage.

Dans un deuxième exemple de l'ensemble de verrouillage 200, le premier élément de verrouillage 213 comprend une tige de verrouillage et le deuxième élément de verrouillage 222 comprend une ouverture de verrouillage. Dans ce cas, dans la configuration d'engagement, l'ouverture de verrouillage reçoit la tige de verrouillage.

Dans la configuration de verrouillage, en réponse à une rotation du raccord 220 par rapport à la base 210 dans une direction de verrouillage prédéterminée, la conduite 221 se trouve au-dessus d'une partie d'extrémité 211, 212 de la base 210, dite partie d'extrémité de verrouillage.

Par ailleurs, le premier élément de verrouillage 213 et le deuxième élément de verrouillage 222 sont en prise l'un avec l'autre de sorte que le raccord 220 et la base 210 sont verrouillés l'un par rapport à l'autre, en direction verticale et horizontale.

On entend par « direction verticale » et « direction horizontale » les directions situées respectivement perpendiculaire et parallèle à la peau du porteur, lorsque le système d'alimentation entérale est en place.

En pratique, le verrouillage du raccord 220 avec la base 210 est tel qu'il forme une rainure d'espacement périphérique ouverte vers l'extérieur qui s'étend entre la surface inférieure du corps de raccord et la surface supérieure de la base 210.

Ainsi, dans la configuration d'engagement, l'ensemble de verrouillage 200 est dans un état verrouillé.

Dans ce cas, le seul moyen de passer de l'état verrouillé à l'état déverrouillé consiste à mettre en rotation le raccord 220 par rapport à la base 210 dans une direction opposée à la direction de verrouillage prédéterminée.

Or, certains porteurs de systèmes d'alimentation entérale, tels que les enfants, bougent beaucoup lorsque le système d'alimentation entérale est en place ; en particulier, la nuit. Dans ce cas, les nombreux mouvements réalisés par le porteur du système d'alimentation entérale peuvent involontairement provoquer le passage de l'état verrouillé à l'état déverrouillé de l'ensemble de verrouillage 200.

Une telle situation peut avoir de graves conséquences sur la santé du porteur du système d'alimentation entérale.

L'un des objectifs de cette invention est de fournir un mécanisme qui permet d'éviter la situation décrite ci-dessus.

Pour cela, l'inventeur propose un capuchon qui, lorsque le l'ensemble de verrouillage 200 est dans l'état verrouillé, permet le verrouillage en rotation de l'ensemble de verrouillage 200.

De cette manière, les mouvements réalisés par le porteur du système d'alimentation entérale ne peuvent plus involontairement provoquer le passage de l'état verrouillé à l'état déverrouillé de l'ensemble de verrouillage 200.

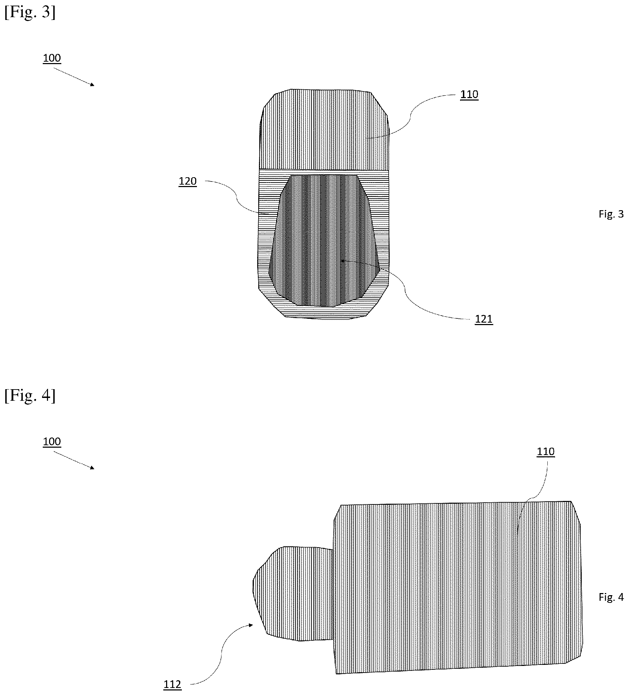

Ainsi, l'invention concerne un capuchon tel qu'illustré sur les figures 2 à 8.

En pratique, le capuchon 100 comprend un corps de capuchon en matériau élastiquement déformable.

En pratique, cela signifie que le corps de capuchon est configuré pour se déformer sous une force et revenir à un état initial non déformé après la suppression de la force.

Dans une mise en œuvre particulière du corps de capuchon, le matériau élastiquement déformable est réalisé dans matériau choisi parmi : un polyéthylène de très basse densité, les élastomères des copolymères propylène/éthylène, des polyéther blocamides, des polyvinyles, des terpolymères d'éthylène, de propylène et d'un diène, EPDM, des polymères de styrène-butadiène séquencés (SBS), des polymères de styrène-éthylène-butadiène séquencés, SEBS-SIS, des polyuréthanes thermoplastiques, les mélanges de polypropylène avec l'un des élastomères choisi parmi les polymères de styrène-éthylène-butadiène séquencés, SEBS-SIS, les terpolymères d'éthylène, de propylène et d'un diène, EPDM, les polymères de styrène-butadiène séquencés, SBS, les caoutchoucs naturels, les élastomères de nitrile, de butyle ou de silicone médical, et toutes combinaisons de ceux-ci.

Toutefois, selon les besoins, on pourra envisager d'utiliser d'autres types de matériaux qui sont élastiquement déformables, et ce, sans nécessiter de modifications substantielles de l'invention.

Par ailleurs, le corps de capuchon comprend un corps de capuchon qui présente une paroi supérieure 110 et une première paroi verticale 120.

La paroi supérieure 110 s'étend entre une extrémité avant et une extrémité arrière selon une direction longitudinale.

On entend par « avant » et « arrière » les parties situées respectivement à gauche et à droite sur la représentation de la [Fig.2].

En outre, la première paroi verticale 120 supporte la paroi supérieure 110 et est raccordée au niveau de l'extrémité arrière de la paroi supérieure 110.

Par ailleurs, la première paroi verticale 120 présente une face avant, une face arrière, une épaisseur, une largeur et une hauteur.

Dans une mise en œuvre particulière du corps de capuchon, celui-ci présente une section en forme de L, comme illustré sur la [Fig.2].

Dans un premier exemple de cette mise en œuvre particulière, la paroi supérieure 110 et la première paroi verticale 120 sont réalisées d'un seul tenant.

Dans un deuxième exemple de cette mise en œuvre, la paroi supérieure 110 et la première paroi verticale 120 sont réalisées en plusieurs morceaux assemblés.

De retour à la [Fig.2], la première paroi verticale 120 comprend une première ouverture de passage 121 qui s'étend sur tout ou partie de la largeur et/ou de la hauteur de la première paroi verticale 120.

En pratique, la première ouverture de passage 121 débouche vers la face avant et vers la face arrière de la première paroi verticale 120.

Dans une mise en œuvre particulière, la largeur de la première ouverture de passage 121 est supérieure à la largeur extérieure de la conduite 221 et également supérieure au diamètre extérieur du tube 230.

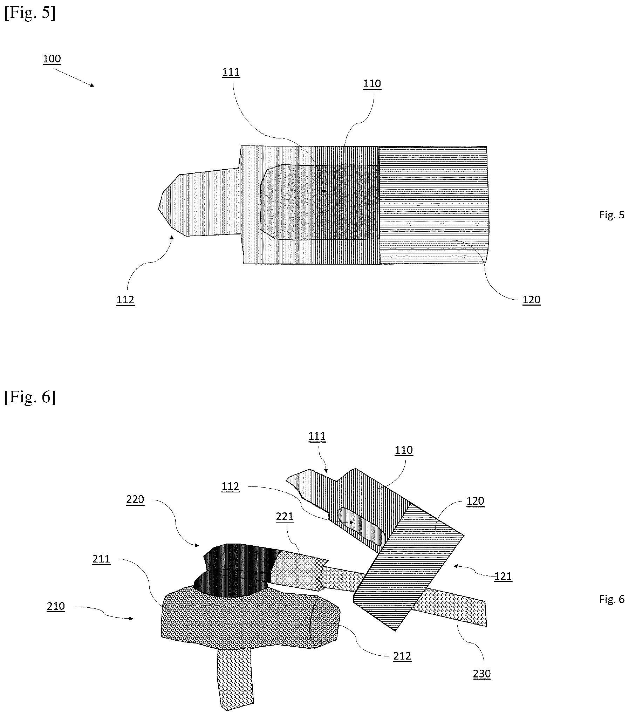

Dans la configuration d'engagement, comme illustré sur la [Fig.6], la première ouverture de passage 121 est destinée à être traversée par le tube 230 .

En pratique, la première ouverture de passage 121 est configurée de sorte que l'on peut faire passer le corps de raccord depuis la face arrière de la première paroi verticale 120 à travers la première ouverture de passage 121 jusqu'à la face avant de la première paroi verticale 120, et ce, de sorte que le tube 230 traverse la première ouverture de passage 121.

Ainsi, comme illustré sur la [Fig.6], si l'on maintient en place le corps de capuchon, il suffit d'exercer une ou plusieurs forces sur le corps de raccord pour déplacer le corps de raccord dans une première direction d'enfilage jusqu'à ce que le tube 230 traverse la première ouverture de passage 121.

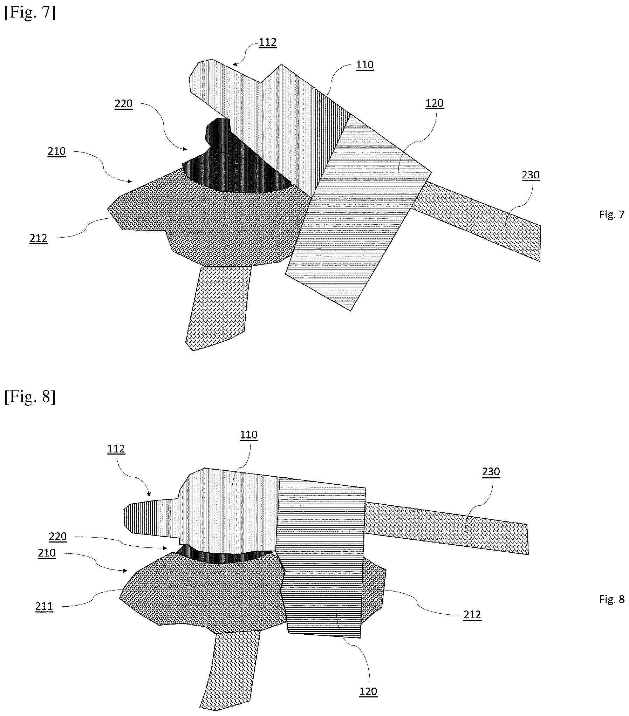

Dans la configuration de verrouillage, comme illustré sur les figures 7 et 8, la première ouverture de passage 121 est destinée à entourer étroitement, au moins, tout ou partie de la conduite 221 et de la partie d'extrémité de verrouillage, de manière à verrouiller en rotation la base 210 et le raccord 220 l'un par rapport à l'autre.

En pratique, si l'on maintient en place le corps de raccord, il suffit d'exercer une ou plusieurs forces sur la première paroi verticale 120 pour déplacer la première paroi verticale 120 dans une deuxième direction d'enfilage opposée à la première direction d'enfilage jusqu'à ce que la première ouverture de passage 121 entoure étroitement tout ou partie de la conduite 221 et de la partie d'extrémité de verrouillage, notamment sous l'effet des propriétés élastiques du corps de capuchon.

Afin de s'assurer du bon positionnement de la première ouverture de passage 121 par rapport à la conduite 221 et la partie d'extrémité de verrouillage, on peut prévoir que la face avant de la première paroi verticale 120 entre en butée contre au moins une partie de la base 210.

Dans un exemple, la largeur de la première ouverture de passage 121, dans son état non déformé, est inférieure à la largeur de partie centrale de la base 210.

Ainsi, lors du déplacement de la première paroi verticale 120 dans la deuxième direction d'enfilage, la première paroi verticale 120 va entrer en butée contre la partie centrale de la base pour signifier le bon positionnement de la première ouverture de passage 121 par rapport à la conduite 221 et la partie d'extrémité de verrouillage.

Afin de renforcer l'interdiction de verrouillage en rotation, on peut prévoir qu'une portion de la partie d'extrémité de verrouillage dépasse au-delà de la face arrière de la première paroi verticale 120.

Dans un exemple, tel qu'illustré sur la [Fig.8], la première paroi verticale 120 est configurée pour que dans la configuration de verrouillage, une portion de la partie d'extrémité de verrouillage dépasse d'une longueur prédéterminée au-delà de la face arrière de la première ouverture de passage 121.

Dans cet exemple, l'épaisseur de la première paroi verticale 120 est sensiblement inférieure à la longueur de la partie d'extrémité de verrouillage.

Par la suite, comme illustré sur la [Fig.5], la paroi supérieure 110 comprend une surface intérieure qui définit un logement 111 creux.

En pratique, dans la configuration de verrouillage, le logement 111 est destiné à recevoir étroitement, par clipsage, tout ou partie de la surface supérieure du corps de raccord, notamment sous l'effet des propriétés élastiques du corps de capuchon.

En particulier, tout ou partie d'une bordure du logement 111 est configurée pour être fixé par clipsage dans la rainure d'espacement périphérique telle que décrite plus haut.

Enfin, on notera que le corps de capuchon est agencé de sorte que la surface supérieure du corps de raccord ne peut pénétrer dans le logement 111 qu'après que la première ouverture de passage 121 entoure tout ou partie de la conduite 221 et de la partie d'extrémité de verrouillage.

Dans une première mise en œuvre de l'invention, tel qu'illustré sur les figures 2 à 8, la paroi supérieure 110 présente une surface extérieure qui comprend une portion de déclipsage 112 qui fait saillie en porte-à-faux vers l'extérieur depuis l'extrémité avant de la paroi supérieure 110.

En outre, la portion de déclipsage 112 présente une surface supérieure et une surface inférieure.

Par ailleurs , dans la configuration de verrouillage, la paroi supérieure 110 est configurée pour être inclinée manuellement sous l'effet d'une force exercée sur la surface inférieure de la portion de déclipsage 112 pour entraîner le déclipsage de la surface supérieure du corps de raccord du logement 111.

En particulier, le déclipsage permet de déloger la bordure du logement 111 de la rainure d'espacement périphérique.

Dans une deuxième mise en œuvre de l'invention, on considère que la première partie d'extrémité 211 et la deuxième partie d'extrémité 212 sont opposées l'une à l'autre.

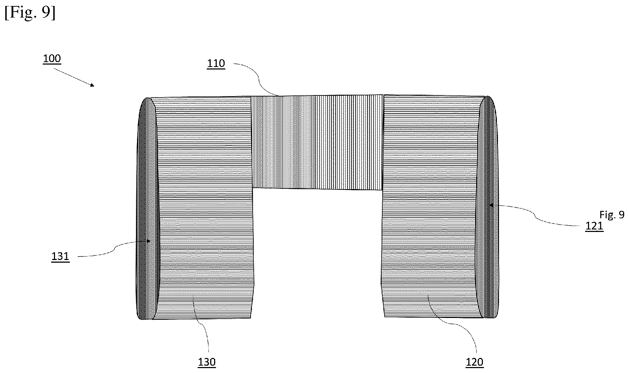

Aussi, tel qu'illustré sur la [Fig.9], le capuchon 100 comprend en outre une deuxième paroi verticale 130 qui supporte la paroi supérieure 110 et est raccordée au niveau de l'extrémité avant de la paroi supérieure 110 de sorte que le corps de capuchon présente une section en forme de U.

Dans un premier exemple de la deuxième mise en œuvre de l'invention, la paroi supérieure 110, la première paroi verticale 120 et la deuxième paroi verticale 130 sont réalisées d'un seul tenant.

Dans un deuxième exemple de la deuxième mise en œuvre de l'invention, la paroi supérieure 110, la première paroi verticale 120 et la deuxième paroi verticale 130 sont réalisées en plusieurs morceaux assemblés.

En outre, la deuxième paroi verticale 130 présente une face avant, une face arrière, une épaisseur, une largeur et une hauteur.

Par ailleurs, la deuxième paroi verticale 130 comprend une deuxième ouverture de passage 131 qui s'étend sur tout ou partie de la largeur et/ou de la hauteur de la deuxième paroi verticale 130.

En pratique, la deuxième ouverture de passage 131 débouche vers la face avant et vers la face arrière de la deuxième paroi verticale 130.

Dans une mise en œuvre particulière, la largeur de la deuxième ouverture de passage 131 est supérieure à la largeur extérieure de la languette 240 et également supérieure à largeur extérieure du couvercle 250.

Dans la configuration d'engagement, la deuxième ouverture de passage 131 est destinée à être traversée par la languette 240.

En pratique, la deuxième ouverture de passage 131 est configurée de sorte que l'on peut faire passer la languette 240 depuis la face arrière de la deuxième paroi verticale 130 à travers la deuxième ouverture de passage 131 jusqu'à la face avant de la deuxième paroi verticale 130, et ce, de sorte que la languette 240 traverse la deuxième ouverture de passage 131.

Ainsi, si l'on maintient en place le corps de capuchon, il suffit d'exercer une ou plusieurs forces sur la languette 240 pour déplacer la languette 240 à travers la deuxième ouverture de passage 131.

Par ailleurs, dans la configuration de verrouillage, la deuxième ouverture de passage 131 est destinée à entourer étroitement, au moins, la partie d'extrémité 211, 212 de la base 210 qui est opposée à la partie d'extrémité de verrouillage, dite extrémité opposée, de manière à verrouiller encore davantage en rotation la base 210 et le raccord 220 l'un par rapport à l'autre.

En pratique, si l'on maintient en place le corps de raccord, il suffit d'exercer une ou plusieurs forces sur la deuxième paroi verticale 130 pour déplacer la deuxième paroi verticale 130 en direction du corps de raccord et de la base 210, jusqu'à ce que la deuxième ouverture de passage 131 entoure étroitement au moins la partie d'extrémité opposée, notamment sous l'effet des propriétés élastiques du corps de capuchon.

Enfin, on notera que le corps de capuchon est agencé de sorte que la deuxième ouverture de passage 131 ne peut entourer la partie d'extrémité opposée qu'après que la surface supérieure du corps de raccord pénètre dans le logement 111.

Afin de s'assurer du bon positionnement de la deuxième ouverture de passage 131 par rapport à la partie d'extrémité opposée, on peut prévoir que la face arrière de la deuxième paroi verticale 130 entre en butée contre au moins une partie de la base 210.

Dans un exemple, la largeur de la deuxième ouverture de passage 131, dans son état non déformé, est inférieure à la largeur de partie centrale de la base 210.

Ainsi, lors du déplacement de la deuxième paroi verticale 130 en direction du corps de raccord et de la base 210, la deuxième paroi verticale 130 va entrer en butée contre la partie centrale de la base pour signifier le bon positionnement de la deuxième ouverture de passage 131 par rapport à la partie d'extrémité opposée.

Afin de renforcer l'interdiction de verrouillage en rotation, on peut prévoir qu'une portion de la partie d'extrémité opposée dépasse au-delà de la face avant de la deuxième paroi verticale 130.

Dans un exemple, la deuxième paroi verticale 130 est configurée pour que dans la configuration de verrouillage, une portion de la partie d'extrémité opposée dépasse d'une longueur prédéterminée au-delà de la face avant de la deuxième ouverture de passage 131.

Dans cet exemple, l'épaisseur de la deuxième paroi verticale 130 est sensiblement inférieure à la longueur de la partie d'extrémité opposée.

Nous avons décrit et illustré l'invention. Toutefois, l'invention ne se limite pas aux formes de réalisations que nous avons présentées. En effet, de nombreuses combinaisons des variantes, alternatives, modes de réalisation et mises en œuvre, peuvent être envisagées sans nécessiter de modifications substantielles de l'invention. Ainsi, un expert du domaine peut déduire d'autres variantes, alternatives, modes de réalisation et mises en œuvre, à la lecture de la description et des figures annexées et en fonction des contraintes économiques, ergonomiques, dimensionnelles à respecter.

L'invention peut faire l'objet de nombreuses variantes et applications autres que celles décrites ci-dessus. En particulier, sauf indication contraire, les différentes caractéristiques structurelles et fonctionnelles de chaque mise en œuvre particulière décrite ci-dessus ne doivent pas être considérées comme combinées et/ou étroitement et/ou inextricablement liées les unes aux autres, mais, au contraire, comme de simples juxtapositions. En outre, les caractéristiques structurelles et/ou fonctionnelles des différents modes de réalisation décrits ci-dessus peuvent faire l'objet en tout ou partie de toute juxtaposition différente ou de toute combinaison différente.

## Revendications

1. Capuchon (100) pour un ensemble de verrouillage (200) d'un système d'alimentation entérale, l'ensemble de verrouillage (200) comprenant une base (210) d'un dispositif de cathéter et un raccord (220) adapté pour être raccordé de manière amovible à la base (210) de sorte que le raccord (220) et la base (210) sont verrouillés l'un par rapport à l'autre, en direction verticale et horizontale, l'ensemble de verrouillage étant configuré pour être verrouillé/déverrouillé par une rotation du raccord (220) par rapport à la base (210),
- la base (210) ayant une première partie d'extrémité (211), une partie centrale et une deuxième partie d'extrémité (212) qui est opposée à la première partie d'extrémité, la première partie d'extrémité et la deuxième partie d'extrémité faisant saillie depuis la partie centrale, la base (210) ayant une surface supérieure et une surface inférieure, la surface supérieure comprenant un premier élément de verrouillage (213),
- le raccord (220) comprenant un corps de raccord et une conduite (221), la conduite (221) définissant un chemin de fluide et étant couplée à un tube (230), le corps de raccord comprenant une surface supérieure et une surface inférieure, la surface inférieure comprenant un deuxième élément de verrouillage (222) qui présente une forme complémentaire à celle du premier élément de verrouillage (213) et qui est configuré pour évoluer entre une configuration d'engagement et une configuration de verrouillage,
- dans la configuration d'engagement, le premier élément de verrouillage (213) et le deuxième élément de verrouillage (222) entrent en prise l'un avec l'autre,
- dans la configuration de verrouillage, en réponse à une rotation du raccord (220) par rapport à la base (210) dans une direction de verrouillage prédéterminée, la conduite (221) se trouve au-dessus d'une partie d'extrémité (211, 212) de la base (210), dite partie d'extrémité de verrouillage, et le premier élément de verrouillage (213) et le deuxième élément de verrouillage (222) sont en prise l'un avec l'autre de sorte que le raccord (220) et la base (210) sont verrouillés l'un par rapport à l'autre, en direction verticale et horizontale,
dans lequel,
le capuchon (100) comprend un corps de capuchon en matériau élastiquement déformable de manière à se déformer sous une force et revenir à un état initial non déformé après la suppression de la force, le corps de capuchon comprenant un corps de capuchon ayant une paroi supérieure (110) et une première paroi verticale (120) qui supporte la paroi supérieure et qui est raccordée au niveau d'une extrémité arrière de la paroi supérieure (110),
- la première paroi verticale (120) comprenant une première ouverture de passage (121) qui s'étend sur tout ou partie d'une largeur et/ou d'une hauteur de la première paroi verticale (120) et qui,
- dans la configuration d'engagement est traversée par le tube (230),
- dans la configuration de verrouillage entoure étroitement, au moins, tout ou partie de la conduite (221) et de la partie d'extrémité de verrouillage, de manière à verrouiller en rotation la base (210) et le raccord (220) l'un par rapport à l'autre,
- la paroi supérieure (110) comprenant une surface intérieure qui définit un logement (111) creux qui est formé dans une épaisseur de la paroi supérieure, de sorte que dans la configuration de verrouillage, le logement (111) creux reçoit étroitement, par clipsage, tout ou partie de la surface supérieure du corps de raccord,
le corps de capuchon étant agencé de sorte que, sous l'effet des propriétés élastiques du corps de capuchon, la surface supérieure du corps de raccord pénètre dans le logement (111) après que la première ouverture de passage (121) entoure tout ou partie de la conduite (221) et de la partie d'extrémité de verrouillage.

2. Capuchon (100) selon la revendication 1, dans lequel le corps de capuchon présente une section en forme de L.

3. Capuchon (100) selon la revendication 2, dans lequel la paroi supérieure (110) présente une surface extérieure qui comprend une portion de déclipsage (112) qui fait saillie en porte-à-faux vers l'extérieur depuis une extrémité avant de la paroi supérieure (110) et qui, dans la configuration de verrouillage, est configurée pour être inclinée manuellement sous l'effet d'une force exercée sur une surface inférieure de la portion de déclipsage (112) pour entraîner le déclipsage de la surface supérieure du corps de raccord du logement (111).

4. Capuchon (100) selon l'une quelconque des revendications 1 à 3, dans lequel la largeur de la première ouverture de passage (121), dans son état non déformé, est inférieure à une largeur de partie centrale de la base (210).

5. Capuchon (100) selon l'une quelconque des revendications 1 à 4, dans lequel la première paroi verticale (120) est configurée pour que dans la configuration de verrouillage, une portion de la partie d'extrémité de verrouillage dépasse d'une longueur prédéterminée au-delà d'une face arrière de la première ouverture de passage (121).

6. Capuchon (100) selon l'une quelconque des revendications 1 à 5, dans lequel la paroi supérieure (110) et la première paroi verticale (120) sont réalisées d'un seul tenant.

7. Capuchon (100) selon l'une quelconque des revendications 1 à 6, comprenant en outre une deuxième paroi verticale (130) qui supporte la paroi supérieure et qui est raccordée au niveau d'une extrémité avant de la paroi supérieure (110) de sorte que le corps de capuchon présente une section en forme de U, la deuxième paroi verticale (130) comprenant une deuxième ouverture de passage (131) qui s'étend sur tout ou partie d'une largeur et/ou d'une hauteur de la deuxième paroi verticale (130) et qui,
- dans la configuration de verrouillage, entoure étroitement, au moins, la partie d'extrémité (211, 212) de la base (210) qui est opposée à la partie d'extrémité de verrouillage, dite extrémité opposée, de manière à verrouiller encore davantage en rotation la base (210) et le raccord (220) l'un par rapport à l'autre,
le corps de capuchon étant agencé de sorte que la deuxième ouverture de passage (131) ne peut entourer la partie d'extrémité opposée qu'après que la surface supérieure du corps de raccord pénètre dans le logement (111).

8. Capuchon (100) selon la revendication 7, dans lequel la largeur de la deuxième ouverture de passage (131), dans son état non déformé, est inférieure à une largeur de partie centrale de la base (210).

9. Capuchon (100) selon l'une quelconque des revendications 7 à 8, dans lequel la paroi supérieure (110), la première paroi verticale (120) et la deuxième paroi verticale (130) sont réalisées d'un seul tenant.

10. Capuchon (100) selon l'une quelconque des revendications 7 à 9, dans lequel la deuxième paroi verticale (130) est configurée pour que dans la configuration de verrouillage, une portion de la partie d'extrémité opposée dépasse d'une longueur prédéterminée au-delà de la face avant de la deuxième ouverture de passage (131).

## Patentansprüche

1. Kappe (100) für eine Verriegelungsanordnung (200) eines enteralen Ernährungssystems, wobei die Verriegelungsanordnung (200) eine Basis (210) einer Kathetervorrichtung und ein Verbindungsstück (220) umfasst, das geeignet ist, lösbar mit der Basis (210) derart verbunden zu sein, dass das Verbindungsstück (220) und die Basis (210) in vertikaler und horizontaler Richtung relativ zueinander verriegelt sind, wobei die Verriegelungsanordnung ausgelegt ist, um durch eine Drehung des Verbindungsstücks (220) relativ zur Basis (210) verriegelt/entriegelt zu werden,
- wobei die Basis (210) einen ersten Endabschnitt (211), einen Mittelabschnitt und einen zweiten Endabschnitt (212) aufweist, der dem ersten Endabschnitt gegenüberliegt, wobei der erste Endabschnitt und der zweite Endabschnitt vom Mittelabschnitt vorstehen, wobei die Basis (210) eine obere Fläche und eine untere Fläche aufweist, wobei die obere Fläche ein erstes Verriegelungselement (213) umfasst,
- wobei das Verbindungsstück (220) einen Verbindungskörper und eine Leitung (221) umfasst, wobei die Leitung (221) einen Fluidweg definiert und mit einem Schlauch (230) verbunden ist, wobei der Verbindungskörper eine obere Fläche und eine untere Fläche umfasst, wobei die untere Fläche ein zweites Verriegelungselement (222) umfasst, das eine zum ersten Verriegelungselement (213) komplementäre Form aufweist und das ausgelegt ist, um sich zwischen einer Eingriffs- und einer Verriegelungskonfiguration zu bewegen,
- wobei in der Eingriffskonfiguration das erste Verriegelungselement (213) und das zweite Verriegelungselement (222) ineinandergreifen,
- wobei in der Verriegelungskonfiguration, als Reaktion auf eine Drehung des Verbindungsstücks (220) relativ zur Basis (210) in einer vorbestimmten Verriegelungsrichtung, sich die Leitung (221) über einem Endabschnitt (211, 212) der Basis (210), bezeichnet als Verriegelungs-Endabschnitt, befindet, und das erste Verriegelungselement (213) und das zweite Verriegelungselement (222) miteinander derart in Eingriff stehen, dass das Verbindungsstück (220) und die Basis (210) in vertikaler und horizontaler Richtung relativ zueinander verriegelt sind,
wobei
die Kappe (100) einen Kappenkörper aus elastisch verformbarem Material umfasst, so dass er sich unter einer Kraft verformt und nach Aufhebung der Kraft in einen unverformten Ausgangszustand zurückkehrt, wobei der Kappenkörper einen Kappenkörper mit einer oberen Wand (110) und einer ersten vertikalen Wand (120) umfasst, die die obere Wand trägt und die im Bereich eines hinteren Endes der oberen Wand (110) verbunden ist,
- wobei die erste vertikale Wand (120) eine erste Durchgangsöffnung (121) umfasst, die sich über die gesamte oder einen Teil einer Breite und/oder Höhe der ersten vertikalen Wand (120) erstreckt und die
- in der Eingriffskonfiguration vom Schlauch (230) durchquert wird,
- in der Verriegelungskonfiguration zumindest die Leitung (221) und den Verriegelungs-Endabschnitt ganz oder teilweise eng umschließt, um die Basis (210) und das Verbindungsstück (220) drehfest miteinander zu verriegeln,
- wobei die obere Wand (110) eine innere Fläche umfasst, die eine hohle Aufnahme (111) definiert, die in einer Dicke der oberen Wand derart ausgebildet ist, dass in der Verriegelungskonfiguration die hohle Aufnahme (111) die gesamte oder einen Teil der oberen Fläche des Verbindungskörpers eng durch Einrasten aufnimmt,
wobei der Kappenkörper derart eingerichtet ist, dass unter der Wirkung der elastischen Eigenschaften des Kappenkörpers die obere Fläche des Verbindungkörpers in die Aufnahme (111) eindringt, nachdem die erste Durchgangsöffnung (121) die Leitung (221) und den Verriegelungs-Endabschnitt ganz oder teilweise umgibt.

2. Kappe (100) nach Anspruch 1, wobei der Kappenkörper einen L-förmigen Querschnitt aufweist.

3. Kappe (100) nach Anspruch 2, wobei die obere Wand (110) eine Außenfläche aufweist, die einen Ausrastabschnitt (112) umfasst, der von einem vorderen Ende der oberen Wand (110) aus freitragend nach außen ragt und die in der Verriegelungskonfiguration ausgelegt ist, um unter der Wirkung einer auf eine untere Fläche des Ausrastabschnitts (112) ausgeübten Kraft manuell geneigt zu werden, um das Ausrasten der oberen Fläche des Verbindungskörpers aus der Aufnahme (111) zu bewirken.

4. Kappe (100) nach einem der Ansprüche 1 bis 3, wobei die Breite der ersten Durchgangsöffnung (121) in ihrem unverformten Zustand kleiner ist als eine Breite eines Mittelabschnitts der Basis (210).

5. Kappe (100) nach einem der Ansprüche 1 bis 4, wobei die erste vertikale Wand (120) ausgelegt ist, damit in der Verriegelungskonfiguration ein Abschnitt des Verriegelungs-Endabschnitts eine Rückseite der ersten Durchgangsöffnung (121) um eine vorgegebene Länge überragt.

6. Kappe (100) nach einem der Ansprüche 1 bis 5, wobei die obere Wand (110) und die erste vertikale Wand (120) einstückig ausgebildet sind.

7. Kappe (100) nach einem der Ansprüche 1 bis 6, umfassend ferner eine zweite vertikale Wand (130), die die obere Wand trägt und im Bereich eines vorderen Endes der oberen Wand (110) derart verbunden ist, dass der Kappenkörper einen U-förmigen Querschnitt aufweist, wobei die zweite vertikale Wand (130) eine zweite Durchgangsöffnung (131) umfasst, die sich über die gesamte oder einen Teil einer Breite und/oder Höhe der zweiten vertikalen Wand (130) erstreckt und die
- in der Verriegelungskonfiguration zumindest den Endabschnitt (211, 212) der Basis (210), der dem Verriegelungs-Endabschnitt, bezeichnet als gegenüberliegendes Ende, ganz oder teilweise derart eng umschließt, um die Basis (210) und das Verbindungsstück (220) noch mehr drehfest miteinander zu verriegeln,
wobei der Kappenkörper derart eingerichtet ist, dass die zweite Durchgangsöffnung (131) den gegenüberliegenden Endabschnitt erst umschließen kann, nachdem die obere Fläche des Verbindungskörpers in die Aufnahme (111) eingedrungen ist.

8. Kappe (100) nach Anspruch 7, wobei die Breite der zweiten Durchgangsöffnung (131) in ihrem unverformten Zustand kleiner ist als eine Breite des Mittelabschnitts der Basis (210).

9. Kappe (100) nach einem der Ansprüche 7 bis 8, wobei die obere Wand (110), die erste vertikale Wand (120) und die zweite vertikale Wand (130) einstückig ausgebildet sind.

10. Kappe (100) nach einem der Ansprüche 7 bis 9, wobei die zweite vertikale Wand (130) ausgelegt ist, damit in der Verriegelungskonfiguration ein Abschnitt des gegenüberliegenden Endabschnitts die Vorderseite der zweiten Durchgangsöffnung (131) um eine vorgegebene Länge überragt.

## Claims

1. A cap (100) for locking assembly (200) of an enteral feeding system, the locking assembly (200) comprising a base (210) of a catheter device and a connector (220) adapted to be removably connected to the base (210) so that the connector (220) and base (210) are locked with each other in vertical and horizontal direction, the locking assembly being configured to be locked/unlocked by rotation of the connector (220) relative to the base (210),
- the base (210) having a first end part (211), a central part and a second end part (212) opposite the first end part, the first end part and second end part protruding from the central part, the base (210) having an upper surface and lower surface, the upper surface comprising a first locking element (213),
- the connector (220) comprising a connector body and a conduit (221), the conduit (221) defining a fluid pathway and being coupled to a tube (230), the connector body comprising an upper surface and lower surface, the lower surface comprising a second locking element (222) having a shape mating with the shape of the first locking element (213) and being configured to move between an engaging configuration and a locking configuration,
- in the engaging configuration, the first locking element (213) and second locking element (222) engage with each other,
- in the locking configuration, in response to rotation of the connector (220) relative to the base (210) in a predetermined locking direction, the conduit (221) lies above an end part (211, 212) of the base (210), called locking end part, and the first locking element (213) and second locking element (222) are engaged with each other to obtain locking together of the connector (220) and base (210) in vertical and horizontal direction,
wherein:
the cap (100) comprises a cap body in elastically deformable material so that it can deform under a force and return to an initial non-deformed state after removal of the force, the cap body comprising a cap body having a top wall (110) and a first vertical wall (120) supporting the top wall and which is connected at a back end of the top wall (110),
- the first vertical wall (120) comprising a first passageway opening (121) extending over all or part of a width and/or height of the first vertical wall (120), and which
- in the engaging configuration, allows the passing of a tube (230),
- in the locking configuration, closely surrounds at least all or part of the conduit (221) and locking end part, to obtain rotational locking together of the base (210) and connector (220),
- the top wall (110) comprising an inner surface defining a hollow housing (111) formed in a thickness of the top wall so that, in the locking configuration, the hollow housing (111) via snap-fit closely receives all or part of the upper surface of the connector body,
the cap body being arranged so that, under the effect of the elastic properties of the cap body, the upper surface of the connector body enters the housing (111) after the first passageway opening (121) surrounds all or part of the conduit (221) and locking end part.

2. The cap (100) according to claim 1, wherein the cap body has an L-shaped cross-section.

3. The cap (100) according to claim 2, wherein the top wall (110) has an outer surface comprising a cantilevered snap-release portion (112) protruding outwardly from a front end of the top wall (110) and which, in the locking configuration, is configured to be manually tilted under the effect of a force applied to a bottom surface of the snap-release portion (112) to release the upper surface of the connector body from the housing (111).

4. The cap (100) according any of claims 1 to **3,** wherein the width of the first passageway opening (121), in the non-deformed state thereof, is smaller than a width of the central part of the base (210).

5. The cap (100) according to any of claims 1 to 4, wherein the first vertical wall (120) is configured so that, in the locking configuration, a portion of the locking end part protrudes over a predetermined length beyond a back face of the first passageway opening (121).

6. The cap (100) according to any of claims 1 to 5, wherein the top wall (110) and the first vertical wall (120) are in one piece.

7. The cap (100) according to any of claims 1 to **6,** additionally comprising a second vertical wall (130) supporting the top wall and which is connected at a front end of the top wall (110) so that the cap body has a U-shaped cross-section, the second vertical wall (130) comprising a second passageway opening (131) extending over all or part of a width and/or height of the second vertical wall (130), and which:
- in the locking configuration, closely surrounds at least the end part (211, 212) of the base (210) opposite the locking end part, called opposite end, to obtain further rotational locking together of the base (210) and connector (220),
the cap body being arranged so that the second passageway opening (131) is only able to surround the opposite end after the upper surface of the connector body enters the housing (111).

8. The cap (100) according to claim 7, wherein the width of the second passageway opening (131), in the non-deformed state thereof, is smaller than a width of the central part of the base (210).

9. The cap (100) according to any of claims 7 to 8, wherein the top wall (110), first vertical wall (120) and second vertical wall (130) are in one piece.

10. The cap (100) according to any of claims 7 to 9, wherein the second vertical wall (130) is configured so that, in the locking configuration, a portion of the opposite end part protrudes over a predetermined length beyond the front face of the second passageway opening (131).
